(19) Europäisches Patentamt
European Patent Office
Office européen des brevets

(11) Veröffentlichungsnummer: **0 440 789 A1**

(12) # EUROPÄISCHE PATENTANMELDUNG
## veröffentlicht nach Art. 158 Abs. 3 EPÜ

(21) Anmeldenummer: **88907443.1**

(51) Int. Cl.[5]: **C07D 227/087**

(22) Anmeldetag: **22.04.88**

(86) Internationale Anmeldenummer:
**PCT/SU88/00093**

(87) Internationale Veröffentlichungsnummer:
**WO 89/10359 (02.11.89 89/26)**

(43) Veröffentlichungstag der Anmeldung:
**14.08.91 Patentblatt 91/33**

(84) Benannte Vertragsstaaten:
**BE CH DE FR GB IT LI**

(71) Anmelder: **2-i MOSKOVSKY GOSUDARSTVENNY MEDITSINSKY INSTITUT IMENI N.I. PIROGOVA**
**ul. Ostrovityanova 1**
**Moscow, 117437(SU)**

(72) Erfinder: **SHIPOV, Alexandr Gennadievich**
**ul. Molodogvardeiskaya, 4-86**
**Moscow, 121467(SU)**
Erfinder: **KRAMAROVA, Evgenia Petrovna**
**ul. Z.Kosmodemyanskoi, 17-2-19**
**Moskovskaya obl. Schelkovo, 141100(SU)**
Erfinder: **ORLOVA, Natalia Andreevna**
**pr. Vernadskogo, 99-1-57**
**Moscow, 117526(SU)**
Erfinder: **BAUKOV, Jury Ivanovich**
**ul. Akademika Anokhina, 34-2-253**
**Moscow, 117602(SU)**
Erfinder: **ZIEMELIS, Kristap Martynovich**
**ul. Muryanu, 48-40**
**Riga, 226004(SU)**

(74) Vertreter: **von Füner, Alexander, Dr. et al**
**Patentanwälte v. Füner, Ebbinghaus, Finck**
**Mariahilfplatz 2 & 3**
**W-8000 München 90(DE)**

(54) **CARBALKOXYMETHYLETHER VON LACTAM-1-ESSIGSÄURE SOWIE HERSTELLUNGSVERFAHREN.**

(57) Die Erfindung bezieht sich auf die organische Chemie. Karbalkoxymethylester der Laktam-1-essigsäure haben folgende allgemeine Formel:

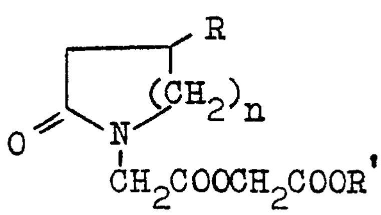

worin bie R = H n = 1 oder 3, bei R = R = Phenyl n = 1 ist und R' für Alkyl steht.

Das Verfahren zur Herstellung der angegebenen Verbindungen besteht darin, dass man Laktame der allgemeinnen Formel

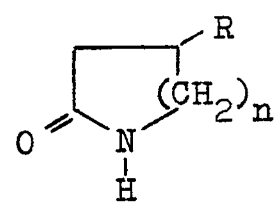

worin bei R = H n = 1 oder 3, bei R = Phenyl n = 1 ist, mit Alkali im Medium von aprotischen Lösungsmitteln bei einer zwischen 70 und 130°C liegenden Temperatur umsetzt, dann dem gewonnenen Gemisch Monohalogenessigsäurealkylester zusetzt und das Endprodukt isoliert.

EP 0 440 789 A1

Die erfindungsgemässen Verbindungen sind Zwischenverbindungen bei der Synthese der bekannten biologisch wirksamen Stoffe.

Gebiet der Technik

Die vorliegende Erfindung bezieht sich auf die organische Chemie und insbesondere auf neue Verbindungen Karbalkoxymethylester der Laktam-1-essigsäure und auf ein Verfahren zur Herstellung derselben. Die erfindungsgemässen Verbindungen stellen Zwischenverbindungen bei der Synthese der bekannten biologisch wirksamen Stoffe mit Peptidbindung zum Beispiel von 2-(2-Oxo-1-pyrrolidinylazetamido)azetamid, 2-(2-Oxo-4-phenyl-1-pyrrolidinylazetamido) azetamid, 2-(2-Oxohexahydro-1-azepinylazetamido)azetamid und von anderen Verbindungen dar.

Zugrundeliegender Stand der Technik

Zur Zeit sind Ester der Laktam-1-essigsäure und verschiedene Verfahren zur Herstellung derselben bekannt. Bekannt ist zum Beispiel ein Verfahren zur Herstellung von Estern der Laktam-1-essigsäure aus Laktamen und Estern der Monohalogenessigsäure unter Verwendung von in Toluol dispergiertem Natriumhydroxid als Methylierungsmittel und anschliessender Azeotropdestillation von Wasser (RO, A, 77162, 77180). Die dabei entstehenden Alkylester werden zur Bildung der Peptidbindung mit den Aminosäurederivaten nicht verwendet. Die aktivierten Ester der Laktam-1-essigsäure, welche an der Bildung der Peptidbindung mit den Aminosäurederivaten teilnehmen, werden aus Laktamen mehrstufig hergestellt.

So ist zum Beispiel bekannt ein Verfahren zur Herstellung von Trichlorphenylester der Laktam-1-essigsäure durch Umsetzung von 1-Trimethylsilyllaktam mit Bromessigsäuretrichlorphenylester in Gegenwart von Propylenoxid (SU, A, 984407) oder ein Verfahren zur Kondensation von N-Hydroxysukzinimid mit Laktam-1-essigsäure in Gegenwart von Dizyklohexylkarbodiimid, welches zur Bildung der entsprechenden Ester führt (DD, A, 215539).

Offenbarung der Erfindung

Die erfindungsgemässen Verbindungen und das Verfahren zur Herstellung derselben sind neu und in der Literatur nicht beschrieben.

Der Erfindung liegt die Aufgabe zugrunde, neue Verbindungen zur Peptidsynthese von biologisch wirksamen Verbindungen nach einer vereinfachten Technologie in einer hohen Ausbeute herzustellen.

Die Aufgabe ist dadurch gelöst worden, dass erfindungsgemäss neue Verbindungen Karbalkoxymethylester der Laktam-1-essigsäure der allgemeinen Formel

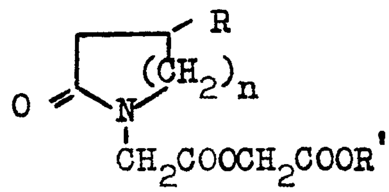

beansprucht werden, worin bei R = H n = 1 oder 3 und bei R = Phenyl n = 1 ist, R' für Alkyl steht.

Die erfindungsgemässen Karbalkoxymethylester der Laktam-1-essigsäure stellen stabile, zähflüssige, farblose oder gelbliche Flüssigkeiten oder niedrigschmelzende weisse kristalline Stoffe dar, die unter vermindertem Druck destilliert werden. Ihre Struktur ist mit Hilfe von Methoden der IR- und NMR-Spektroskopie, durch Elementaranalyse und chemische Umsetzungen derselben nachgewiesen.

Die erfindungsgemässen Karbalkoxymethylester der Laktam-1-essigsäure sind aktivierte Ester und können im Unterschied zu den bekannten Alkylestern der Laktam-1-essigsäure zur Bildung der Peptidbindung mit Aminosäurederivaten unter schonenden Bedingungen bei der Synthese der bekannten biologischenwirksamen Verbindungen zum Beispiel von 2-(2-Oxo-1-pyrrolidinylazetamido)azetamid, 2-(2-Oxo-4-phenyl-1-pyrrolidinylazetamido)azetamid, 2-(2-Oxohexahydro-1-azepinylazetamido)azetamid und anderen Verbindungen zur Verwendung kommen. Die Verwendung der erfindungsgemässen Verbindungen macht es möglich, die Bildung der Peptidbindung ohne Teilnahme von konventionellen Kondensationsmitteln wie Dizyklohexylkarbodiimid, die in diesen Fällen eingesetzt werden, zu bewirken sowie die Ausbeute an Peptidsyntheseprodukten zu steigern.

Das Verfahren zur Herstellung von den erfindungsgemässen Verbindungen besteht erfindungsgemäss darin, dass man Laktame der allgemeinen Formel

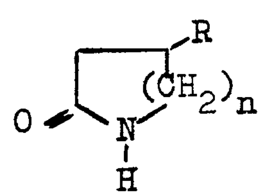

worin bei R = H n = 1 oder 3 und bei R = Phenyl, n = 1 ist, zur Umsetzung mit Alkali im Medium von aprotischen Lösungsmitteln bei einer zwischen 70 und 130°C liegenden Temperatur bringt, dann dem gewonnenen Gemisch Monohalogenessisäurealkylester zusetzt und das Endprodukt isoliert. Zwecks Vereinfachung der Verfahrenstechnologie ist es vorteilhaft, dals Alkali Kaliumhydroxid und als aprotische Lösungsmittel Dimethylsulfoxid

oder sein Gemisch mit Toluol bzw. Benzol zu verwenden. Zur Erhöhung der Endproduktausbeute ist Äthylchlorazetat oder Äthylbromazetat als Monohalogenessigsäurealkylester zweckmässigerweise zu verwenden.

Das erfindungsgemässe Verfahren zur Herstellung von Karbalkoxymethylestern der Laktam-1-essigsäure aus Laktamen erfolgt im Vergleich zu den bekannten Verfahren zur Herstellung der anderen aktivierten Ester der Laktam-1-essigsäure einstufig und gestattet hohe Ausbeuten an Endprodukten zu erreichen.

Beste Ausführungsform der Erfindung

Das erfindungsgemässe Verfahren wird wie folgt durchgeführt.

Man erhitzt ein Laktam mit Alkali in einem aprotischen Lösungsmittel bei einer zwischen 70 und 130°C liegenden Temperatur, bis das Alkali völlig dispergiert wird. Als Alkali kann man Natrium- oder Kaliumhydroxid und als aprotisches Lösungsmittel beispielsweise Dimethylsulfoxid oder seine Gemische mit Benzol bzw. Toluol einsetzen. Dem Reaktionsgut setzt man danach einen Monohalogenessigsäureester zum Beispiel Äthylchlorazetat oder Äthylbromazetat zu und isoliert das Endprodukt. Das Isolieren des Endprodukts erfolgt durch Fraktionieren des Reaktionsgemisches unter vermindertem Druck oder durch Lösungsmittelextraktien des Endprodukts.

Die Ausbeute an Endprodukt erreicht 75% der Theorie.

Zum besseren Verstehen der vorliegenden Erfindung werden folgende Beispiele zur Ausführung des Verfahrens zur Herstellung der erfindungsgemässen Verbindungen angeführt.

Beispiel 1

In einen Dreihalskolben, versehen mit einem Rührwerk, Tropftrichter und Rückflusskühler, bringt man 25,5 g (0,3 Mol) 2-Pyrrolidon, 33,6 g (0,6 Mol) Kaliumhydroxid und 100 ml Dimethylsulfoxid ein. Das Gemisch wird im Wasserbad bei einer Temperatur von 70°C unter Umrühren so lange erhitzt, bis das Alkali völlig dispergiert wird. Dem Gemisch setzt man danach 73,5 g (0,6 Mol) Äthylchlorazetat tropfenweise zu und lässt bei Raumtemperatur für mehrere Stunden stehen. Man filtriert das Gemisch, trennt durch Fraktionieren des Filtrats 38 g (55,3%) Karboäthoxymethylester der (2-Oxo-1-pyrrolidinyl)-essigsäure mit Siedepunkt von 197 bis 200°C (11 Torr), $n_D^{20}$ = 1,4700.

IR-Spektrum ( $\nu$, $cm^{-1}$, Lösung $ClCH_2CH_2Cl$): 1730 (C = 0,Ester), 1675 (C = 0, Laktam). H-NMR-Spektrum ( $\delta$ , ppm. $CDCl_3$): 1,26 Triplett, ($CH_3$, J 7 Hz), 2,09 Multiplett und 2,36 Multiplett ($CH_2CH_2CO$ des Zyklus, J 7 Hz), 3,50 Multiplett ($NCH_2$ des Zyklus, J,6,6 Hz), 4,16 Singulett ($CH_2N$), 4,20 Quartett ($OCH_2CH_3$, J 7 Hz), 4,36 Singulett ($OCH_2CO$).

Gefunden in %: C 52,25; H 6,66; N 6,19. $C_{10}H_{15}NO_5$

Berechnet in %: C 52,39; H 6,59; N 6,11.

Beispiel 2

Aus 25,5 g (0,3 Mol) 2-Pyrrolidon, 16,8 g (0,3 Mol) Kaliumhydroxid, 100 ml Dimethylsulfoxid und 36,8 g (0,3 Mol) Äthylchlorazetat erhält man analog zu dem in Beispiel 1 beschriebenen 17,6 g (25,6%) Karbäthoxymethylester der (2-Oxo-1-pyrrolidinyl)-essigsäure mit Siedepunkt von 197 bis 200°C (10 Torr), $n_D^{20}$ = 1,4705.

Beispiel 3

Man verfährt analog zu dem in Beispiel 1 beschriebenen.

Man nimmt 25,5 g (0,3 Mol) 2-Pyrrolidon, 50,4 g (0,9 Mol) Kaliumhydroxid in 200 ml Dimethylsulfoxid und 115 g (0,94 Mol) Äthylchlorazetat. Nach dem Halten des Reaktionsguts bei Raumtemperatur setzt man diesem 600 ml Wasser zu und extrahiert dreimal je 100 ml Dichloräthan. Die vereinigten Extrakte werden eingedampft, durch Fraktionieren des Rückstands erhält man 46,9 g (68%) Karbäthoxymethylester der (2-Oxo-1-pyrrolidinyl)-essigsäure mit Siedepunkt von 197 bis 200°C (10 Torr), $n_D^{20}$ =1,4703.

Beispiel 4

Das Gemisch von 25,5 g (0,3Mol) 2-Pyrrolidon, 33,6 g (0,6 Mol) Kaliumhydroxid, 150 ml Dimethylsulfoxid und 100 ml Benzol lässt man unter Verwendung vom Rückflusskühler beim Umrühren so lange sieden, bis das Alkali völlig dispergiert wird. Dem Gemisch setzt man tropfenweise 73,5 g (0,6 Mol) Äthylchlorazetat zu und lässt weitere 2 Stunden sieden, bringt danach die Temperatur auf Raumtemperatur. Der Salzniederschlag wird abfiltriert. Durch Fraktionieren des Gemisches trennt man 35,7 g (52%) Karbäthoxymethylester der (2-Oxo-1-pyrrolidinyl) essigsaure mit Seidepunkt von 195 bis 198°C (9 Torr), $n_D^{20}$ = 1,4706.

Beispiel 5

Ein Gemisch von 25,5 g (0,3 Mol) 2-Pyrrolidon, 40 g (1 Mol) Natriumhydroxid in 200 ml Dimethylsulfoxid rührt man bei einer Temperatur von 90°C so lange um, bis das Alkali völlig dispergiert wird, setzt dann dem Gemisch tropfenweise 122,5 g (1 Mol) Äthylchlorazetat zu und lässt bei Raumtemperatur für mehrere Stunden stehen. Dem Gemisch setzt man 600 ml Wasser zu und extrahiert dreimal je 130 ml Chloroform, die vereinigten Extrakte werden eingedampft, durch Fraktionieren des Rückstands trennt man 39 g (56,8%) Karbäthoxymethylester der (2-Oxo-1-pyrrolidinyl)essigsäure mit Siedepunkt von 195 bis 199°C (10 Torr), $n_D^{20}$ = 1,4705.

Beispiel 6

Ein Gemisch von 25,5 g (0,3 Mol) 2-Pyrrolidon, 33,6 g (0,6 Mol) Kaliumhydroxid und 200 ml Dimethylsulfoxid rührt man bei einer Temperatur von 70°C so lange um, bis das Alkali völlig dispergiert wird, dann setzt man tropfenweise 100,2 g (0,6 Mol) Äthylbromazetat zu und lässt für mehrere Stunden bei Raumtemperatur stehen. Das Gemisch verdünnt man mit 600 ml Wasser und extrahiert dreimal je 100 ml Chloroform. Die vereinigten Extrakte werden eingedampft, durch Fraktionieren des Rückstands trennt man 42,6 g (62%) Karbäthoxymethylester der (2-Oxo-1-pyrrolidinyl)essigsäure mit Siedepunkt von 196 bis 198°C (9 Torr), $n_D^{20}$ = 1,4701.

Beispiel 7

In 500 ml Dimethylsulfoxid und 80,5 g (0,5 Mol) 4-Phenyl-2-pyrrolidon werden 70 g (1,25 Mol) Kaliumhydroxid bei einer zwischen 110 und 115°C liegenden Temperatur völlig dispergiert. Dem Gemisch setzt man tropfenweise 153 g (1,25 Mol) Äthylchlorazetat zu und lässt bei Raumtemperatur für 24 Stunden stehen. Das Gemisch wird mit 1,5 Liter Wasser verdünnt und zweimal je 200 ml Benzol extrahiert. Die vereinigten Extrakte werden eingedampft, durch Fraktionieren des Rückstands erhält man 79 g (52%) Karbäthoxymethylester der (2-Oxo-4-phenyl-1-pyrrolidinyl)essigsäure mit Siedepunkt von 220 bis 223°C (1,5 Torr), $n_D^{20}$ =1,5215.

IR-Spektrum ( $\nu$, $cm^{-1}$, $CCl_4$): 1745 (C = 0, Ester), 1700 (C = 0, Laktam).

H'-NMR-Spektrum ( $\delta$, ppm, $CDCl_3$): 1,27 Triplett ($CH_3$, J 6 Hz), 2,75 Multiplett ($CH_2CO$ des Zyklus), 3,70 Multiplett ($CHCH_2N$ des Zyklus), 4,20 Quartett ($OCH_2CH_3$, J 6 Hz), 4,25 Singulett ($NCH_2CO$), 4,66 Singulett ($OCH_2CO$), 7,29 Singulett ($C_6H_5$).

Kermagnetisches $^{13}C$-Resonanzspektrum ( $\delta$ , ppm, $CDCl_3$): 14,05, 37,20, 38,30, 43,70, 54,50, 61.13, 61.45, 126.81, 127.01, 128.76, 142.29, 167.13, 168.11, 174.35. Gefunden in %: C 62,86; H 6,22; N 4,62. $C_{16}H_{19}NO_5$.Berechnet in %: C 62,94; H 6,27; N 4,59.

Beispiel 8

In einem Gemisch aus 300 ml Dimethylsulfoxid und 40 g (0,248 Mol) 4-Phenyl-2-pyrrolidon werden bei einer Temperatur von 100°C 45 g (0,8 Mol) Kaliumhydroxid dispergiert, dann setzt man tropfenweise 120 g (0,98 Mol) Äthylchlorazetat zu und lässt bei Raumtemperatur für mehrere Stunden stehen. Das Gemisch wird mit 800 Liter Wasser verdünnt und dreimal je 100 ml Benzol extrahiert. Die vereinigten Extrakte werden eingedampft, durch Fraktionieren des Rückstands trennt man 55,7 g (75,6%) Karbäthoxymethylester der (2-Oxo-4-phenyl-1-pyrrolidinyl) essigsäure mit Siedepunkt von 210 bis 214°C (1 Torr), $n_D^{20}$ = 1,5210.

Beispiel 9

Ein Gemisch von 80,5g(0,5 Mol) 4-Phenyl-2-pyrrolidon, 70 g (1,25 Mol) Kaliumhydroxid, 500 ml Dimethylsulfoxid und 150 ml Toluol lässt man unter Umrühren bei der Verwendung des Rückflusskühlers bei einer Temperatur von 130°C sieden, bis das Alkali völlig dispergiert wird. Dann setzt man dem Gemisch 153 g (1,25 Mol) Äthylchlorazetat tropfenweise zu und lässt bei Raumtemperatur für 24 Stunden stehen. Das Gemisch wird mit 1,2 Liter Wasser verdünnt und zweimal je 100 ml Toluol extrahiert, durch Fraktionieren der Extrakte trennt man 73,2 g (47%) Karbäthoxymethylester der (2-Oxo-4-phenyl-1-pyrrolidinyl)essigsäure mit Siedepunkt von 210 bis 214°C (1 Torr), $n_D^{20}$ = =1,5212.

Beispiel 10

Einer bei einer Temperatur von 110 bis 115°C hergestellten Dispersion von 30 g (0,75 Mol) Natriumhydroxid in 250 ml Dimethylsulfoxid und 40,3 g (0,25 Mol) 4-Phenyl-2-pyrrolidon setzt man 92 g (0,75 Mol) Äthylchlorazetat zu, erhitzt das Gemisch bei einer Temperatur von 90°C innerhalb von 2 Stunden und bringt die Temperatur auf Raumtemperatur. Das mit 750 ml Wasser verdünnte Gemisch wird dreimal je 70 ml Benzol extrahiert. Durch Fraktionieren der Extrakte trennt man 42 g (55%) Karbäthoxymethylester der (2-Oxo-4-phenyl-1-pyrrolidinyl)essigsäure mit Siedepunkt von 211 bis 214°C (1 Torr), $n_D^{20}$ =1.5210.

Beispiel 11

Einer bei einer Temperatur von 100°C hergestellten Dispersion von 28 g (0,5 Mol) Kaliumhydroxid in 200 ml Dimethylsulfoxid und 40,3 g (0,25 Mol) 4-Phenyl-2-pyrrolidon setzt man tropfenweise 83,5 g (0,5 Mol) Äthylbromazetat zu. Nach 24 Stunden verdünnt man das Gemisch mit 600 ml Wasser und extrahiert zweimal je 100 ml Benzol. Durch Fraktionieren von Benzolextrakten trennt man 46 g (60%) Karbäthoxymethylester der (2-Oxo-4-phenyl-1-pyrrolidinyl)essigsäure mit Siedepunkt von 210 bis 213°C (1 Torr), $n_D^{20}$ = 1,5212.

Beispiel 12

Ein Gemisch von 33,9 g (0,3 Mol) ε -Kaprolaktam, 33,6 g (0,6 Mol) Kaliumhydroxid in 100 ml Dimethylsulfoxid rührt man unter Erhitzen auf eine Temperatur von 100°C so lange um, bis das Alkali völlig dispergiert wird, setzt dann dem Gemisch tropfenweise 73,5 g (0,6 Mol) Äthylchlorazetat zu und lässt bei Raumtemperatur für mehrere Stunden stehen. Das Gemisch verdünnt man mit 400 ml Wasser und extrahiert dreimal je 100 ml Chloroform. Durch Fraktionieren von Extrakten trennt man 38,9 g (50,5 %) Karbäthoxymethylester der (2-Oxohexahydro-1-azepinyl)essigsäure mit Siedepunkt von 210 bis 213°C (12 Torr), Schmelzpunkt von 33,5 bis 35°C (Diäthylester).

IR-Spektrum ( ν , $cm^{-1}$, $CHCl_3$): 1745 (C - 0, Ester), 1635 (C = 0, Laktam).

H'-NMR-Spektrum ( δ, ppm, $CCl_4$): 1,23 Triplett ($CH_3$, J 6 Hz), 1,69 Multiplett ($3CH_2$ des Zyklus), 2,42 Multiplett ($CH_2Co$ des Zyklus), 3,41,Multiplett ($CH_2N$ des Zyklus), 4,12 Quartett ($CH_2O$, J 6 Hz), 4,18 Singulett ($NCH_2CO$), 4,58 Singulett ($OCH_2CO$).

Gefunden in %: C 55,90, H 7,49, N 5,41. $C_{12}H_{19}NO_5$

Berechnet in %: C 56,02, H 7,44, N 5,44

Beispiel 13

Aus 22,6 g (0,2 Mol) ε -Kaprolaktam, 39,2 g (0,7 Mol) granuliertem Kaliumhidroxid, 200 ml Dimethylsulfoxid und 85,8 g (0,7 Mol) Äthylchlorazetat erhält man analog zu dem in Beispiel 12 beschriebenen 30 g (58,4%) Karbäthoxymethylester der (2-Oxo-hexahydro-1-azepinyl)essigsäure mit Siedepunkt von 155 bis 157°C (1 Torr), Schmelzpunkt von 34 bis 35°C (Diäthylester).

Industrielle Anwendbarkeit

Die erfindungsgemässen Karbalkoxymethylester der Laktam-1-essigsäure werden bei der Synthese von biologisch wirksamen Stoffen wie 2-(2-Oxo-1-pyrrolidinylazetamido)azetamid, 2-(2-Oxohexahydro-1-azepinylazetamido) azetamid, 2-(2-Oxo-4-phenyl-1-pyrrolidinylazetamido) azetamid und anderen Verbindungen verwendet, die gegenüber Prozessen, verbunden mit der Gedächtnisverbesserung und der Schutzwirkung beim Sauerstoffmangel, wirksam sind.

**Patentansprüche**

1. Karbalkoxymethylester der Laktam-1-essigsäure der allgemeinen Formel

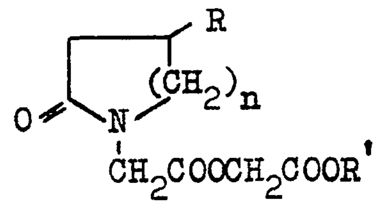

worin bei R = H n = 1 oder 3 und bei R = Phenyl n = 1 ist, R' für Alkyl steht.

2. Verfahren zur Herstellung von Karbalkoxymethylestern der Laktam-1-essigsäure nach Anspruch 1, dadurch **gekennzeichnet**, dass man Laktame der allgemeinen Formel

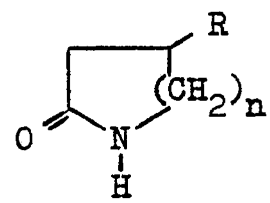

worin bei R = H n = 1 oder 3 und bei R = Phenyl n = 1 ist, zur Umsetzung mit Alkali im Medium eines aprotischen Lösungsmittels bei einer zwischen 70 und 130°C liegenden Temperatur bringt, dann dem egewonnenen Gemisch Monohalogenessisäurealkylester zusetzt und das Endprodukt isoliert.

3. Verfahren nach Anspruch 2, dadurch **gekennzeichnet**, dass als Alkali Kalium- bzw. Natriumhydroksid verwendet wird.

4. Verfahren nach Anspruch 2 bis 3, dadurch **gekennzeichnet**, dass als aprotisches Lösungsmittel Dimethylsulfoxid oder das Gemisch desselben mit Toluol oder Benzol dient.

5. Verfahren nach Anspruch 2 bis 4, dadurch **gekennzeichnet**, dass als Monohalogenessigsäurealkylester Äthylchlorazetat oder Äthylbromazetat verwendet wird.

# INTERNATIONAL SEARCH REPORT

International Application No PCT/SU 88/00093

**I. CLASSIFICATION OF SUBJECT MATTER** (if several classification symbols apply, indicate all) [6]

According to International Patent Classification (IPC) or to both National Classification and IPC

Int. Cl.$^4$ - C 07 D 227/087

**II. FIELDS SEARCHED**

Minimum Documentation Searched [7]

| Classification System | Classification Symbols |
| --- | --- |
| Int.Cl.$^4$ | C 07 D 207/27, 223/IO, 227/087 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
| --- | --- | --- |
| A | GB, B, 1583163 (HOECHST AKTIENGESELLSCHAFT) 21 January 1981 (21.01.81), see claims 1,8a<br>& FR, BI, 2372806, 29.02.80<br>IT, A, 1086223, 28.05.85<br>-- | 1,2 |
| A | US, A, 4341790 (A.NATTERMANN & CIE, GmbH), 27 July 1982 (27.07.82), see column 1, lines 11-20<br>& FR, BI, 2458544, 05.08.83<br>JP, A, 56-2960, 13.01.81<br>GB, A, 2053909, 11.02.81<br>BE, A, 883791, 01.10.80<br>DE, C2, 2924011, 08.04.82 | 1 |
| A | US, A, 4650878 (HOFFMANN - LA ROCHE INC.) 17 March 1987 (17.03.87), see column 5 lines 66-68, column 6 lines 5-15<br>& FR, BI,2515179, 18.01.85<br>GB, B, 2110207, 04.09.85<br>DE, AI,3227649, 10.02.83<br>JP, A, 58-24558,14.02.83 | 2 |

.../...

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
| --- | --- |
| 10. November 1988 (10.11.88) | 13. January 1989 (13.01.89) |
| **International Searching Authority** | **Signature of Authorized Officer** |
| ISA/SU | |

International Application No. PCT/SU 88/00093

**III. DOCUMENTS CONSIDERED TO BE RELEVANT (CONTINUED FROM THE SECOND SHEET)**

| Category * | Citation of Document, with indication, where appropriate, of the relevant passages | Relevant to Claim No |
|---|---|---|
| A | US, A, 4416818 (THE DOW CHEMICAL COMPANY), 22 November 1983 (22.11.83), see claim 1 | 2 |